# EUROPÄISCHE PATENTSCHRIFT

(11) **EP 3 124 391 B1**
(45) Veröffentlichungstag und Bekanntmachung des Hinweises auf die Patenterteilung: **26.09.2018**
(21) Anmeldenummer: 16161697.4
(22) Anmeldetag: 22.03.2016
(51) Int. Cl.: B65D 19/00, B65D 19/44, A61L 2/26, A61B 50/20, A61B 50/30, A61B 50/33, A61B 50/34, F16B 37/04

(54) **TRÄGERANORDNUNG**
SUPPORT ASSEMBLY
SYSTEME DE SUPPORT

(30) Priorität: 30.07.2015 AT 2252015 U
(43) Veröffentlichungstag der Anmeldung: 01.02.2017
(73) Patentinhaber: FRIES PLANUNGS- UND MARKETINGGESELLSCHAFT m.b.H., 6832 Sulz (AT)
(72) Erfinder: Göbl, Otmar, 6844 Altach (AT)
(74) Vertreter: Fechner, Thomas

(56) Entgegenhaltungen:
- EP-A1- 2 860 126
- EP-A1- 2 860 127
- DE-U1-202012 103 711
- DE-U1-202014 102 215
- US-A- 4 077 300
- US-A1- 2013 118 938

## Beschreibung

Die vorliegende Erfindung betrifft eine Trägeranordnung gemäß des Oberbegriffs des Patentanspruchs 1.

Sowohl beim gewerblichen Geschirrspülen als auch bei der industriellen Teilereinigung sowie bei der Sterilisierung von medizinischen Geräten und dergleichen, werden gattungsgemäße Trägeranordnungen benötigt, um die zu reinigenden bzw. zu sterilisierenden Teile bzw. das Geschirr während des Reinigungs- bzw. Sterilisierungsprozesses geeignet zu lagern.

Darüber hinaus können gattungsgemäße Trägeranordnungen auch sonst in der, insbesondere industriellen, Logistik zum Transport und/oder zur Lagerung von Bauteilen, Geschirr und dergleichen verwendet werden. Eine Trägeranordnung ist aus der DE 20 2014 102 215 U1, Fig. 12 und 15 bekannt. Der Befestigungssockel der dort gezeigten Haltepins wird im Kreuzungsbereich von zwei Gitterstäben an diesen klemmend befestigt. Dies hat zur Folge, dass insgesamt vier Öffnungen benötigt werden, um einen Befestigungssockel an der gitterförmigen Struktur klemmend zu befestigen. Die praktische Erfahrung zeigt, dass diese Art der Befestigung des Befestigungssockels an der gitterförmigen Struktur relativ unstabil ist, sodass es in der Praxis dazu kommen kann, dass der Befestigungssockel aus Versehen von der gitterförmigen Struktur gelöst wird.

Die DE 20 2014 102 215 U1 offenbart in Fig. 13 noch eine zweite Art von Befestigungssockeln, welche in Form einer Art Bajonettverschluss ausgeführt sind. Um diese in einer Trägerplatte befestigen zu können, benötigt die Trägerplatte aber speziell an diese Form angepasste Öffnungen, wie sie in Fig. 14 der DE 20 2014 102 215 U1 gezeigt sind. Diese Öffnungen sind nicht mehr rechteckförmig und daher gattungsfremd. Sie haben den Nachteil, dass solche Öffnungen relativ platzeinnehmend sind, sodass die Öffnungen relativ weit voneinander beabstandet sein müssen, wodurch die Flexibilität in der Positionierung des Befestigungssockels in der gitterförmigen Struktur eingeschränkt ist. Außerdem sind solche gattungsfremden gitterförmigen Strukturen mit nicht rechteckförmigen Öffnungen oft relativ anfällig bezüglich der Anlagerung von Verschmutzungen und dergleichen.

In der EP 2 860 126 A1 und der EP 2 860 127 A1 sind gattungsgemäße Trägeranordnungen gezeigt, wobei die gitterförmige Struktur durch sich kreuzende in verschiedenen Ebenen angeordnete Drähte gebildet wird. Die US 2013/0118938 A1 zeigt gattungsfremden Stand der Technik, da dort die Öffnungen im Gitter nicht rechteckförmig sind.

Aufgabe der Erfindung ist es, bei einer Trägeranordnung der oben genannten Art mit einer gitterförmigen Struktur mit rechteckförmigen, insbesondere quadratischen, Öffnungen eine möglichst platzsparende aber dennoch stabil ausführbare Art der Befestigung des Befestigungssockels in der gitterförmigen Struktur vorzuschlagen.

Hierzu schlägt die Erfindung eine Trägeranordnung gemäß Patentanspruch 1 vor.

Es ist ein Grundgedanke, einen Befestigungssockel in einer einzelnen rechteckförmigen, insbesondere quadratischen, Öffnung der gitterförmigen Struktur klemmend zu befestigen. In anderen Worten wird für die klemmende Befestigung des Befestigungssockels nur eine einzelne der rechteckförmigen, insbesondere quadratischen, Öffnungen benötigt. Hierdurch ist es bei Bedarf z.B. möglich, in direkt zueinander benachbarten rechteckförmigen, insbesondere quadratischen, Öffnungen jeweils einen Befestigungssockel klemmend zu befestigen. Hierdurch können die Befestigungssockel sehr dicht aneinander angeordnet werden, falls dies für den jeweiligen Anwendungsfall gewünscht wird. Dies gibt eine maximale Freiheit in der Wahl der Öffnung und damit der Position auf der Trägerplatte, in der der jeweilige Befestigungssockel klemmend befestigt werden soll. Man ist also sehr flexibel in der Bestückung der Trägeranordnung. Außerdem hat sich überraschenderweise gezeigt, dass bei der klemmenden Befestigung eines Befestigungssockels in nur einer einzelnen der rechteckförmigen, insbesondere quadratischen, Öffnungen, eine sehr stabile Befestigung erreicht werden kann.

Um Fehlinterpretationen zu vermeiden, wird der Vollständigkeit halber darauf hingewiesen, dass die Erfindung nicht bedeutet, dass ein Befestigungssockel nur in einer einzigen der rechteckförmigen, insbesondere quadratischen, Öffnungen klemmend befestigt werden kann und in sonst keiner anderen Öffnung. Es ist vielmehr bei der Erfindung insbesondere vorgesehen, dass ein einziger Befestigungssockel aufgrund der bevorzugt jeweils gleichen Ausformung der rechteckförmigen, insbesondere quadratischen, Öffnungen, in einer Vielzahl von verschiedenen dieser Öffnungen befestigt werden kann. Es ist aber eben gemäß der Erfindung so, dass eine einzelne dieser Öffnungen ausreicht, um den Befestigungssockel darin und damit an der Trägerplatte klemmend zu befestigen.

Erfindungsgemäße Trägeranordnungen sind, wie oben bereits ausgeführt, für die Lagerung und/oder den Transport und/oder die Reinigung von Geschirr oder anderen Gegenständen geeignet und/oder vorgesehen. Insbesondere können sie beim gewerblichen Geschirrspülen und/oder in der industriellen Teilereinigung eingesetzt werden. Auch bei der Sterilisierung von z.B. medizinischem Gerät können erfindungsgemäße Trägeranordnungen besonders gut eingesetzt werden. Auch die insbesondere industrielle Lagerung und der insbesondere industrielle Transport von Geschirr und anderen Gegenständen zählt zu den Aufgabengebieten, bei denen erfindungsgemäße Trägeranordnungen eingesetzt werden können bzw. für die diese vorgesehen sein können.

Mit der Erfindung ist ein sehr flexibles modulares System geschaffen, mit dem die Trägeranordnung an die jeweilige Aufgabenstellung, also insbesondere an die in und/oder auf ihr zu lagernden Gegenstände wie z.B. das Geschirr oder die industriellen Bauteile oder die zu sterilisierenden Gerätschaften anpassbar ist. So können zum Einen die Befestigungssockel an den gewünschten Positionen an der Trägerplatte befestigt werden. Zum anderen können an den Befestigungssockeln unterschiedlichste Stütz- oder sonstige Strukturen befestigt werden. Hierbei können die genannten Stütz- oder sonstigen Strukturen sowohl einstückig als auch über eine, vorzugsweise zerstörungsfrei lösbare, Verbindungseinrichtung wie z.B. ein Gewinde oder dergleichen am Befestigungssockel fixiert bzw. befestigt sein oder werden. Besonders bevorzugt ist vorgesehen, dass der Befestigungssockel eine zerstörungsfrei lösbare Verbindungseinrichtung, vorzugsweise in Form einer Gewindebuchse, zur Befestigung einer Stützstruktur oder einer sonstigen Struktur am Befestigungssockel aufweist. Die Gewindebuchse kann z.B. aus Metall gefertigt sein. Bei den Stütz- oder sonstigen Strukturen kann es sich z.B. um Stifte, Stege, Wände, Griffe, gekreuzte, tannenbaumförmige, mehrfingerige oder stabförmige Steher oder auch Steher, die der Stapelung von mehreren Trägeranordnungen übereinander dienen, handeln. Grundsätzlich können unterschiedlichste Strukturen an den Befestigungssockeln befestigt werden, sodass die Trägeranordnung je nach Bedarf an die jeweilige Anwendung angepasst werden kann. Mit einer entsprechenden wandförmigen Struktur ist insbesondere eine Ausgestaltung der Trägeranordnung als Korb möglich.

Die Trägerplatte kann die gitterförmige Struktur und auch andere nicht gitterförmige Bereiche aufweisen. Die Trägerplatte kann aber auch vollständig als gitterförmige Struktur ausgebildet sein. Bei den rechteckförmigen, insbesondere quadratischen Öffnungen kann es sich um vollständig durch die Trägerplatte hindurchgehende Durchgangsöffnungen aber auch um Sacklöcher oder dergleichen handeln. Die Gitterstäbe können sehr unterschiedlich ausgeformt sein. Es kann sich z.B. um rippen- und/oder stegförmige Strukturen oder dergleichen handeln. Die Erfindung sieht vor, dass die die Öffnungen umschließenden Gitterstäbe jeweils zumindest einen Wulst zur klemmenden Befestigung des Befestigungssockels in der Öffnung aufweisen. Bei der Wulst handelt es sich um einen im Querschnitt gegenüber dem restlichen Gitterstab verbreiterten bzw. erweiterten Bereich. Die Wulst kann aber muss nicht im Querschnitt zumindest bereichsweise kreisrund ausgebildet sein. Sie kann auch andere Formen wie z.B. eine Pilzkopfstrukture oder dergleichen aufweisen. An der Wulst können z.B. auch einzelne oder mehrere Hinterschnitte angeformt sein, welche die klemmende Befestigung des Befestigungssockels in der Öffnung unterstützen. Die Wulst ist jedenfalls günstigerweise auf einer, eine Oberfläche der Trägerplatte bildenden Seite der Gitterstäbe ausgebildet. Es ist vorgesehen, dass alle Wülste der Trägerplatte in einer gemeinsamen Ebene enden bzw. in anderen Worten eine gemeinsame Ebene ausbilden. In wiederum anderen Worten stehen somit alle Wülste gleich weit über die sonstigen Bereiche der Gitterstäbe vor, sodass ihre von den sonstigen Bereichen der Gitterstäbe wegweisenden Enden alle in einer gemeinsamen Ebene liegen.

An die Wulst, vorzugsweise an alle Wülste, sind in bevorzugten Ausgestaltungsformen Rippen und/oder stegförmige Strukturen der Gitterstäbe angeformt. Vorzugsweise handelt es sich hierbei um ein einstückiges Anformen der Rippen und/oder stegförmigen Strukturen an die Wülste. Die Rippen und/oder stegförmigen Strukturen der Gitterstäbe stehen günstigerweise alle auf der selben Seite der Trägerplatte von den Wülsten ab. Mit solchen Rippen und/oder stegförmigen Strukturen kann die Stabilität bzw. Tragfähigkeit der Trägerplatte wesentlich verbessert werden.

Die Kantenlänge der rechteckförmigen, insbesondere quadratischen, Öffnungen der gitterförmigen Struktur liegt günstigerweise im Bereich von 4 mm (Millimeter) bis 35 mm, besonders bevorzugt von 6 mm bis 18 mm.

Die Erfindung sieht vor, dass der Befestigungssockel vier paarweise einander gegenüberliegende Rastzungen aufweist.

Die Rastzungen hintergreifen zur Befestigung des Befestigungssockels in der Öffnung jeweils die Wulst eines der die Öffnung umschließenden Gitterstäbe. An den Rastzungen sind Hinterschnitte angeordnet, die die klemmende Befestigung des Befestigungssockels in der jeweiligen Öffnung unterstützen bzw. stabiler machen. Die Rastzungen weisen Hinterschnitte zum Hintergreifen eines Wulstes auf. Des Weiteren kann vorgesehen sein, dass zur Vereinfachung einer elastischen Deformation der Rastzungen zwischen zwei jeweils zueinander benachbarten Rastzungen, vorzugsweise jeweils, ein Spalt angeordnet ist.

Es ist auch vorgesehen, dass der Befestigungssockel eine Platte aufweist, wobei zwischen den Hinterschnitten der Rastzungen und der Platte die Wulst, vorzugsweise spielfrei, einklemmbar oder eingeklemmt ist, wobei die Hinterschnitte aller Rastzungen des Befestigungssockels gleich weit von der Platte distanziert sind. Bevorzugt ist auch vorgesehen, dass die paarweise einander gegenüberliegenden Rastzungen, vorzugsweise in Richtung voneinander weg, elastisch vorgespannt sind. Unter elastischer Vorspannung ist dabei insbesondere zu verstehen, dass die Rastzungen, wenn sie aus einer Ausgangslage ausgelegt werden, mittels elastischer Rückstellkräfte beaufschlagt werden, welche so gerichtet sind, dass sie die Rastzungen in Richtung hin zu ihrer Ausgangslage vorspannen. In der Ausgangslage selbst müssen die Rastzungen dabei nicht elastisch vorgespannt sein.

Im Sinne einer optimalen Anpassung an die rechteckige bzw. insbesondere quadratische Form der Öffnungen sehen bevorzugte Varianten der Erfindung vor, dass der Befestigungssockel vier Rastzungen aufweist und diese Rastzungen zumindest bereichsweise entlang der Seiten eines gedachten Rechtecks, insbesondere Quadrats, verlaufen. Um den Befestigungssockel in der Öffnung klemmend zu befestigen und/oder aus dieser klemmenden Befestigung wieder zu lösen, sehen bevorzugte Varianten vor, dass der Befestigungssockel, vorzugsweise im Bereich zwischen den Rastzungen, zumindest eine Ausnehmung für den Eingriff eines Drehwerkzeugs aufweist. Die Form der Ausnehmung kann dabei an spezielle Drehwerkzeuge wie z. B. Schraubenzieher, Inbusschlüssel und dergleichen angepasst sein. Es kann sich also z.B. um schlitz- oder kreuzschlitzförmige Ausnehmungen oder inbusförmige Ausnehmungen handeln.

Eine andere Art der Ausnehmungen kann aber auch zur Aufnahme eines Spreizstiftes oder einer Spreizschraube dienen. Diese Ausnehmung zur Aufnahme eines Spreizstiftes oder einer Spreizschraube ist besonders bevorzugt zwischen den Rastzungen des Befestigungssockels ausgeführt. Sie kann dazu dienen, dass ein in die Ausnehmung eingeführter Spreizstift oder eine darin eingeschraubte Spreizschraube verhindert, dass die Rastzungen noch ausgelenkt werden können. Hierdurch kann der Befestigungssockel besonders fest und stabil in der Öffnung klemmend befestigt werden. Es kann somit vorgesehen sein, dass ein Spreizstift oder eine Spreizschraube in einer Ausnehmung des Befestigungssockels zwischen Rastzungen des Befestigungssockels aufgenommen ist.

Bei der Art und Weise, wie der Befestigungssockel in der Öffnung eingebracht wird und damit auch bei der Art der Ausgestaltungsform des Befestigungssockels gibt es verschiedene Varianten. Eine erste Gruppe dieser Varianten sieht vor, dass der Befestigungssockel ausschließlich durch ein, vorzugsweise lineares, Eindrücken des Befestigungssockels, vorzugsweise seiner Rastzungen, in die Öffnung klemmend an der Gitterstruktur befestigbar oder befestigt ist. In anderen Worten wird bei diesen Varianten der Befestigungssockel einfach nur weit genug in die Öffnung eingedrückt. In bevorzugten Varianten kommt es dabei zu einer elastischen Auslenkung der Rastzungen an den Wülsten der Gitterstäbe. In der Endstellung, in der der Befestigungssockel klemmend in der Öffnung befestigt ist, hintergreifen dann die Rastzungen günstigerweise die Wulst.

Bei einer anderen Gruppe von erfindungsgemäßen Befestigungssockeln kann hingegen aber auch vorgesehen sein, dass der Befestigungssockel, vorzugsweise ausschließlich, durch ein, vorzugsweise lineares, Einführen des Befestigungssockels, vorzugsweise seiner Rastzungen, in die Öffnung und ein anschließendes Drehen des Befestigungssockels in der Öffnung klemmend an der gitterförmigen Struktur befestigbar oder befestigt ist. Diese Art von Befestigungssockel bildet also eine Art Bajonettverschluss, welcher allerdings im Unterschied zum eingangs genannten Stand der Technik eben in rechteckförmigen, insbesondere quadratischen,

Öffnungen befestigt werden kann. Diese Art von Befestigungssockel ist besonders verschleißarm und damit dauerhaft haltbar bzw. einsatzfähig. Insbesondere bei diesen Varianten ist günstigerweise vorgesehen, dass zwischen den Rastzungen des Befestigungssockels Zwischenseiten vorgesehen sind, welche von der Drehachse, um die der Befestigungssockel in der Öffnung gedreht wird, weniger weit entfernt sind als die Rastzungen selbst. Es kann somit vorgesehen sein, dass zwischen zwei jeweils benachbarten Rastzungen sich jeweils eine Zwischenseite des Befestigungssockels befindet, wobei die Zwischenseiten in einem geringeren Abstand als die Rastzungen von einer Drehachse des Befestigungssockels angeordnet sind. Die Rastzungen und die Zwischenseiten können, in einer Draufsicht gesehen, eine achteckige Struktur ergeben. Diese Struktur ist günstigerweise achsensymmetrisch und/oder punktsymmetrisch. Diese Zwischenseiten können z.B. beim Einführen des Befestigungssockels in die Öffnung parallel zu den Gitterstäben ausgerichtet sein. Durch Drehen des Befestigungssockels in der Öffnung kommen dann die Rastzungen des Befestigungssockels zum Eingriff an den Gitterstäben bzw. besonders bevorzugt an deren Wülsten, woraus sich dann die klemmende Befestigung des Befestigungssockels in der Öffnung an der gitterförmigen Struktur ergibt. Zum Lösen solcher Befestigungssockel aus der klemmenden Befestigung können die Befestigungssockel entweder weitergedreht oder entgegen der Eindrehrichtung zurückgedreht werden. Anschließend können die Befestigungssockel wieder aus den Öffnungen herausgezogen werden. Die Zwischenseiten liegen dabei günstigerweise wieder parallel zu den Gitterstäben.

Bei erfindungsgemäßen Trägeranordnungen können sowohl die Trägerplatte als auch die Befestigungssockel grundsätzlich auch aus Metall, Keramik oder anderen Werkstoffen hergestellt sein. Besonders bevorzugte Ausgestaltungsformen der Erfindung sehen aber vor, dass die Trägerplatte und/oder der Befestigungssockel zumindest zum Teil, vorzugsweise vollständig, aus Kunststoff ausgebildet ist bzw. sind. Solche Trägerplatten und/oder Befestigungssockel lassen sich z.B. aus thermoplastischem Kunststoff, besonders bevorzugt als Spritzgussteile, günstig herstellen.

Weitere Merkmale und Einzelheiten bevorzugter Ausführungsvarianten der Erfindung werden nachfolgend anhand der Figurenbeschreibung erläutert. Es zeigen:
Fig. 1 eine perspektivische Ansicht auf ein erfindungsgemäßes Ausführungsbeispiel einer Trägeranordnung;
Fig. 2 eine Draufsicht auf dieses Ausführungsbeispiel;
Fig. 3 eine Ansicht von unten auf einen Teilbereich dieses Ausführungsbeispiels;
Fig. 4 einen Längsschnitt durch einen Teilbereich dieses Ausführungsbeispiels;
Fig. 5 bis 9 Darstellungen zu einem ersten Ausführungsbeispiel einer ersten Art von Befestigungssockeln für diese Trägeranordnung;
Fig. 10 eine Darstellung zur Erläuterung der Art der Befestigung der in den Fig. 5 bis 9 dargestellten Art der Befestigungssockel in der gitterförmigen Struktur;
Fig. 11 bis 15 eine zweite Art von Befestigungssockeln gemäß der Erfindung und
Fig. 16 bis 20 verschiedene Beispiele von Stütz- oder sonstigen Strukturen, wie sie an erfindungsgemäßen Befestigungssockeln realisiert sein können.

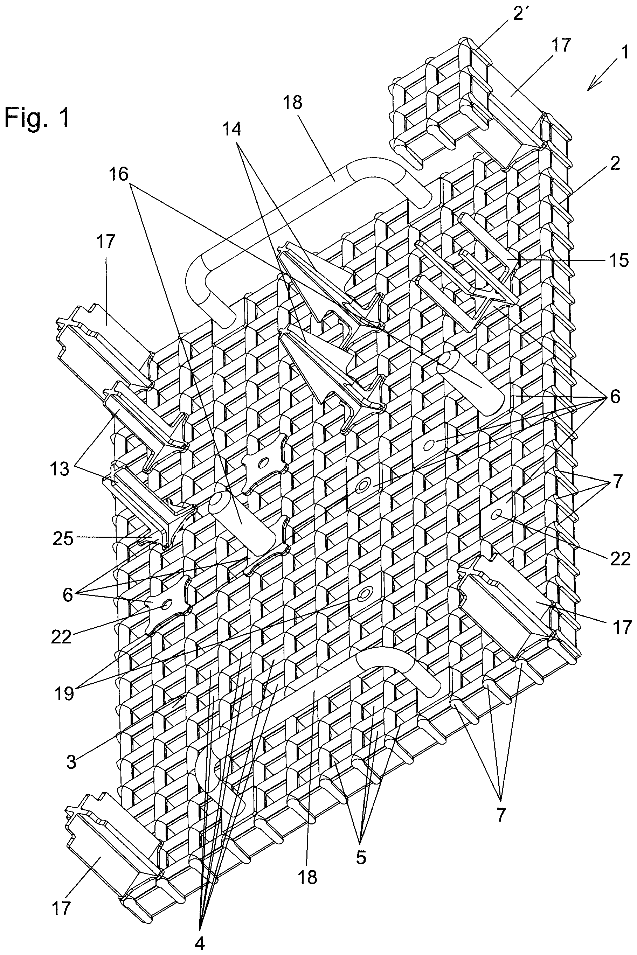

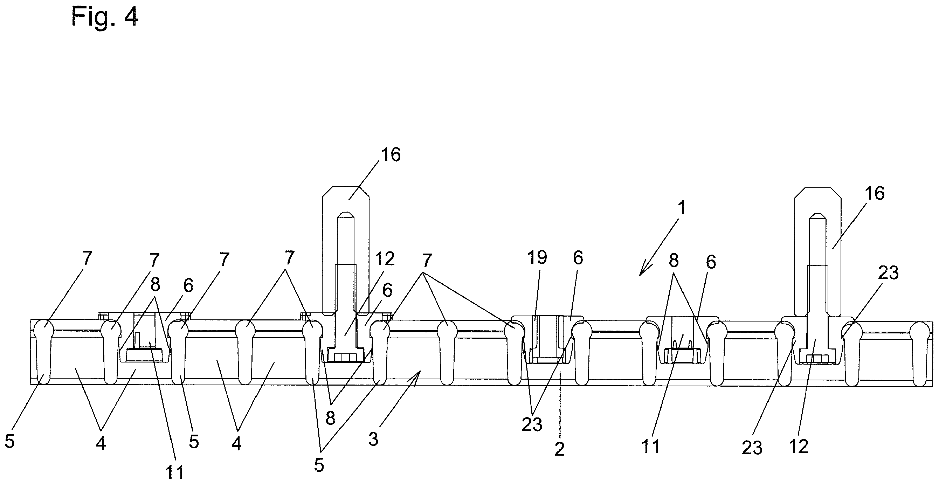

Die Trägerplatte 2 der hier in Fig. 1 dargestellten Trägeranordnung 1 weist in diesem Ausführungsbeispiel quadratische Öffnungen 4 auf, welche jeweils von entsprechend quadratisch angeordneten Gitterstäben 5 umschlossen sind. Die Öffnungen 4 dieser Gitterstruktur 3 dieses Ausführungsbeispiels sind als Durchgangslöcher ausgebildet und haben alle dieselbe Größe. Dies ist günstigerweise auch in anderen bevorzugten Ausgestaltungsformen der Trägeranordnung 1 so gewählt. Letzteres hat den Vorteil, dass die Befestigungssockel 6 in allen Öffnungen 4 klemmend befestigbar sind. Erfindungsgemäß wird jeder der hier gezeigten Befestigungssockel 6 in einer einzelnen der hier quadratisch ausgeformten Öffnungen 4 klemmend befestigt. An den Befestigungssockeln 6 können einstückig oder lösbar verschiedenste Stütz- oder sonstige Strukturen bzw. Bauteile befestigt sein. Gezeigt sind in diesem Ausführungsbeispiel z.B. die Griffe 18, welche mittels erfindungsgemäßer Befestigungssockel 6 an der Trägerplatte 2 klemmend befestigt sind. Darüber hinaus tragen die verschiedenen Befestigungssockel 6 in diesem Ausführungsbeispiel auch verschiedenste Stützstrukturen, wie z.B. die gekreuzten Steher 13, die tannenbaumförmigen Steher 14, die mehrfingerigen Steher 15 und auch die stabförmigen Steher 16. Die gekreuzten, tannenbaumförmigen und mehrfingeringen Steher 13, 14, 15 sind dabei jeweils einstückig an den entsprechenden Befestigungssockel 6 angeformt. Die stabförmigen Steher 16 sind, wie dies in Fig. 4 zu sehen ist, mittels je einer der Spreizschrauben 12 am jeweiligen Befestigungssockel 6 angeschraubt. Einzelne der hier dargestellten Befestigungssockel 6 tragen eine Gewindebuchse 19 in sich. An dieser Gewindebuchse können dann beliebig andere Bauteile durch Festschrauben befestigt werden. Sowohl die Trägerplatte 2 als auch die Befestigungssockel 6 sind in bevorzugten Ausgestaltungsformen günstigerweise aus Kunststoff ausgeführt. Die Alternativen hierzu sind eingangs bereits genannt. Die Gewindebuchsen 19 bestehen jedoch bevorzugt aus Metall. Anstelle der Gewindebuchsen 19 können die Befestigungssockel 6 aber auch einfache Befestigungslöcher 22 aufweisen, in denen dann in einer geeigneten Art und Weise entsprechende Stütz- oder sonstige Strukturen befestigt werden können. Abweichend vom hier gezeigten Ausführungsbeispiel können auch wandförmige Strukturen über entsprechende Befestigungssockel 6 klemmend in der Trägerplatte 2 befestigt werden, z.B. um entsprechende Körbe auszubilden.

In Fig. 1 sind an den vier Ecken der Trägerplatte 2 mittels entsprechender Befestigungssockel 6 Stapelsteher 17 vorgesehen. Diese dienen dazu, dass auf ihnen und damit auf der gezeigten Trägeranordnung 1 eine weitere Trägerplatte 2' einer weiteren Trägeranordnung 1 aufgestapelt werden kann. Dies ist im rechten oberen Eck von Fig. 1 angedeutet.

In der perspektivischen Darstellung gemäß Fig. 1 sind auch gut die hier im Schnitt zumindest bereichsweise kreisförmig ausgebildeten Wülste 7 der Gitterstäbe 5 zu sehen. Die Wülste 7 dienen in den hier gezeigten Ausführungsbeispielen der Befestigung der Befestigungssockel 6 in der jeweiligen Öffnung 4.

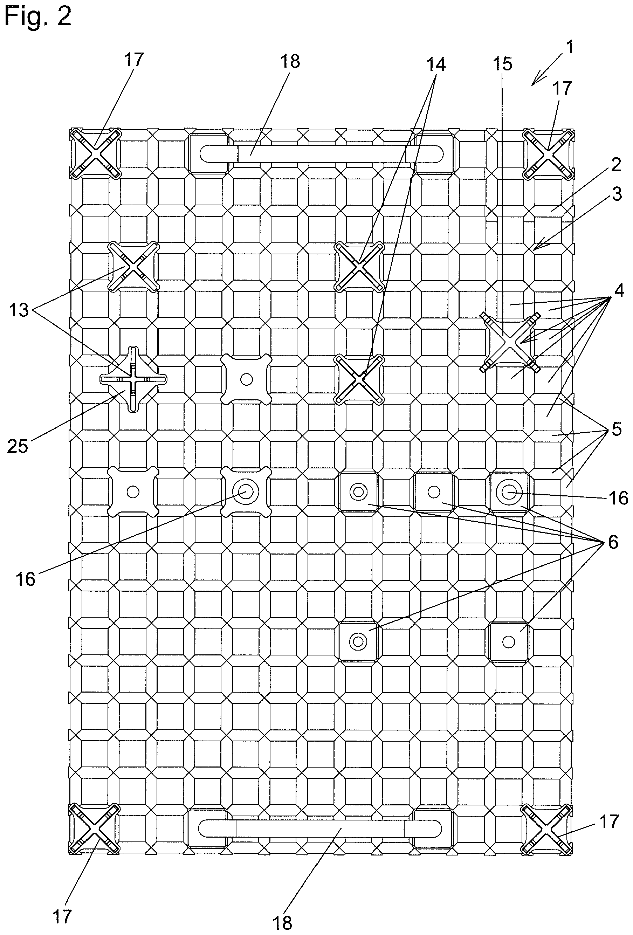

Fig. 2 zeigt eine Draufsicht auf die Trägeranordnung 1 gemäß Fig. 1, wobei allerdings im rechten oberen Rand die in Fig. 1 angedeutete aufgestapelte weitere Trägerplatte 2' weggelassen ist. In Fig. 2 sieht man besonders gut, dass jeder der Befestigungssockel 6 in einer einzelnen der hier quadratischen Öffnungen 4 klemmend befestigt ist.

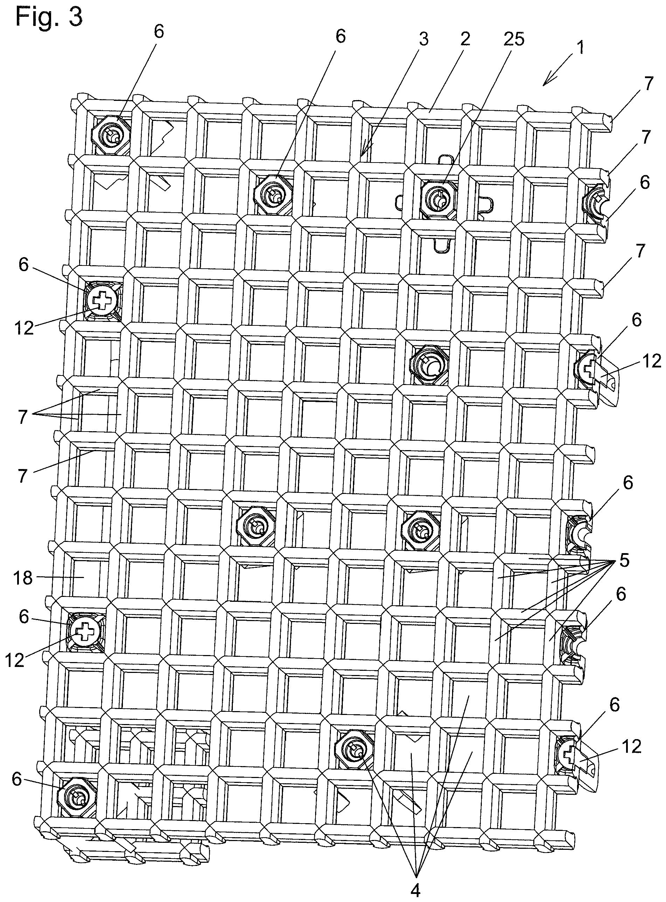

Fig. 3 zeigt eine Ansicht von unten auf einen Teilbereich der Trägeranordnung 1 aus Fig. 1.

In den Fig. 1 bis 3 sind alle bis auf einen der Befestigungssockel 6 in der Stellung eingezeichnet, in der sie in der jeweiligen quadratischen Öffnung 4 klemmend befestigt sind. Die einzige Abweichung hiervon bildet der mit dem Bezugszeichen 25 versehene Befestigungssockel. Dieser ist, wie weiter hinten noch im Detail erläutert, zwar bereits in die Öffnung 4 eingeführt, aber in dieser noch nicht klemmend befestigt.

In der Schnittdarstellung gemäß Fig. 4 ist zunächst gut die Ausbildung der Wülste 7 an den Gitterstäben 5 an einer der Oberflächen der Trägerplatte 2 zu sehen. Weiters ist auch gut zu sehen, wie die Rastzungen 8 der verschiedenen Befestigungssockel 6 in den gezeigten Ausführungsbeispielen mit ihren Hinterschnitten 23 die Wülste 7 hintergreifen, um so den jeweiligen Befestigungssockel 6 erfindungsgemäß in einer einzelnen der Öffnungen 4 klemmend zu befestigen. Die Rastzungen 8 sind jeweils entsprechend elastisch in der Richtung vorgespannt, in der sie die Wulst 7 hintergreifen.

Alle hier gezeigten Ausführungsbeispiele von Befestigungssockeln 6 weisen zwischen den Rastzungen 8 Ausnehmungen 11 auf, in die ein Spreizstift oder hier eine Spreizschraube 12 eingebracht werden kann. Die Spreizschrauben 12, wie auch die hier nicht gezeigten Spreizstifte, stützen die Rastzungen 8 so ab, dass sie aus ihrer Klemmstellung nicht mehr ausgelenkt werden können, sodass der jeweilige Befestigungssockel 6 besonders fest klemmend in der jeweiligen Öffnung 4 befestigt ist. Die Spreizschrauben 12 können, wie hier auch gezeigt, gleichzeitig dazu verwendet werden, entsprechende Stützstrukturen oder andere Bauteile am Befestigungssockel 6 zu befestigen. In Fig. 4 ist dies anhand der mittels der Spreizschrauben 12 am jeweiligen Befestigungssockel 6 befestigten stabförmigen Steher 16 beispielhaft illustriert.

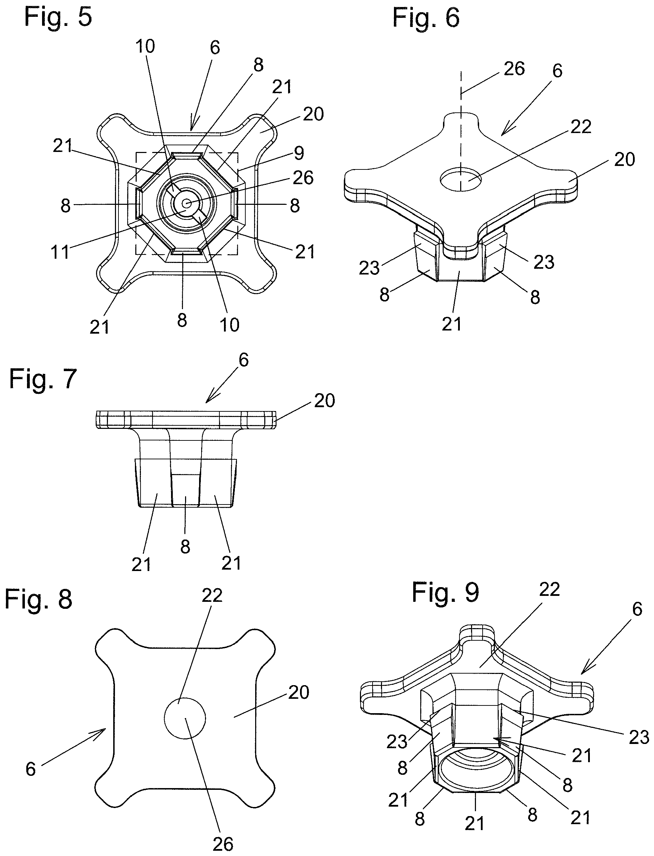

Die Fig. 5 bis 9 zeigen ein Ausführungsbeispiel einer ersten Art von Befestigungssockel 6, welcher durch ein Einführen des Befestigungssockels 6 in die Öffnung 4 und ein anschließendes Drehen des Befestigungssockels 6 in der Öffnung 4 klemmend an der gitterförmigen Struktur 3 befestigbar ist. Gedreht wird der Befestigungssockel 6 hierzu um die Drehachse 26. Wie besonders gut in der Ansicht von unten gemäß Fig. 5 zu sehen, weist der Befestigungssockel 6 vier paarweise einander gegenüberliegende Rastzungen 8 auf. Die jeweils einander paarweise gegenüberliegenden Rastzungen 8 sind in der oben genannten Art und Weise elastisch vorgespannt. Die vier Rastzungen 8 verlaufen zumindest bereichsweise entlang der Seiten eines gedachten Quadrats 9, welches in Fig. 5 ebenfalls eingezeichnet ist. Zwischen zwei jeweils benachbarten Rastzungen 8 befindet sich eine Zwischenseite 21, welche in einem geringeren Abstand als die Rastzungen 8 von der Drehachse 26 angeordnet ist. Die Rastzungen 8 und die Zwischenseiten 21 ergeben in bevorzugten Ausgestaltungsformen, wie der hier gezeigten, eine insgesamt achteckige, und hier auch achsensymmetrische und auch punktsymmetrische Struktur.

Zwischen den Rastzungen 8 befindet sich die Ausnehmung 11, in die ein Spreizstift oder eine Spreizschraube 12 eingeschraubt werden kann, um die Rastzungen 8 in ihrer klemmenden Stellung abzustützen bzw. zu fixieren. Weiters weist der Befestigungssockel 6 gemäß der Fig. 5 bis 9 auch Ausnehmungen 10 für den Eingriff eines Drehwerkzeugs auf. In dem hier gezeigten Ausführungsbeispiel bilden die beiden Ausnehmungen 10 einen Schlitz für einen Schraubenzieher. Dieser kann dazu verwendet werden, den Befestigungssockel 6 um die Drehachse 26 zu drehen, um den Befestigungssockel 6 entweder klemmend in der Öffnung 4 zu befestigen oder auch um diese klemmende Befestigung wieder zu lösen.

In den perspektivischen Ansichten gemäß der Fig. 6 und 9 ist auch noch der jeweilige Hinterschnitt 23 an den Rastzungen 8 gut zu sehen. Mit diesem Hinterschnitt 23 hintergreifen die Rastzungen 8 die Wülste 7, wie dies in Fig. 4 gezeigt ist.

In den hier gezeigten Ausführungsbeispielen weist der Befestigungssockel 6 auch noch eine Platte 20 auf. Diese dient in den hier gezeigten Ausführungsbeispielen als eine Art Widerlager, so dass zwischen den Rastzungen 8 und der Platte 20 die jeweilige Wulst 7, bevorzugt spielfrei, eingeklemmt werden kann. Am Befestigungsloch 22 können verschiedenste Stütz- oder sonstige Strukturen oder dergleichen befestigt werden.

Wie dies in Fig. 4 auch gut zu sehen ist, ist in bevorzugten Ausführungsbeispielen der Erfindung vorgesehen, dass im in einer Öffnung 4 befestigten Zustand des Befestigungssockels 6 die Gitterstäbe 5 auf einer Seite der gitterförmigen Struktur 3 über den jeweiligen Befestigungssockel 6 überstehen. Hierdurch wird erreicht, dass die Befestigungssockel 6 auf dieser Seite der Trägerplatte 2 zwischen den Gitterstäben 5 verborgen sind und auf dieser Seite der Trägerplatte 2 nicht über diese hinwegstehen. Günstigerweise handelt es sich dabei um die Seite der Trägerplatte 2, die den Wülsten 7 entgegengesetzt angeordnet ist. Hierdurch wird erreicht, dass die Befestigungssockel 6 in ihrem in einer Öffnung 4 der Trägerplatte 2 befestigten Zustand nicht nach unten hin über die Trägerplatte 2 überstehen. Dies ermöglicht es z.B. die Trägerplatte 2 über einen ebenen Untergrund zu schieben, ohne dass die Befestigungssockel 6 mit diesem Untergrund kollidieren können.

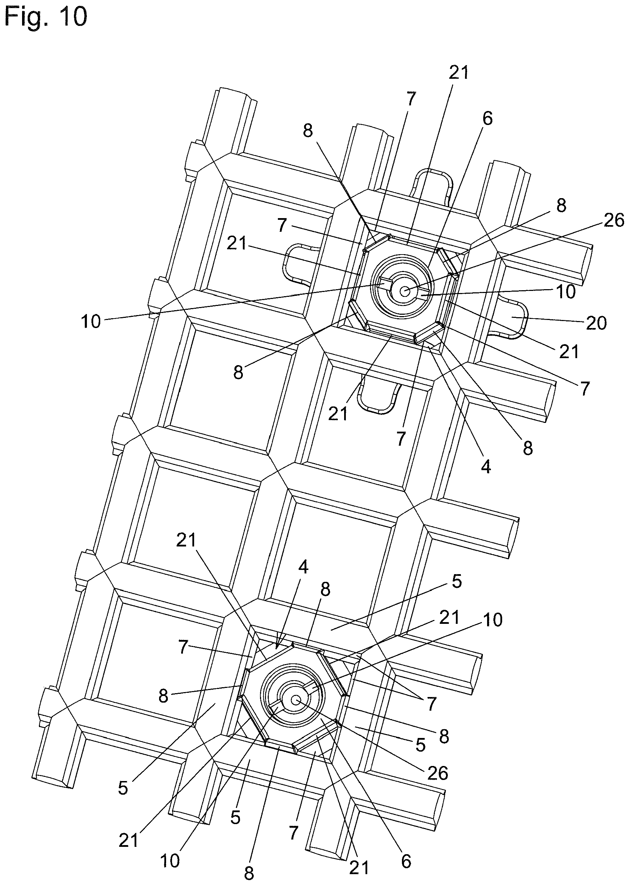

Fig. 10 dient dazu, die Art der Befestigung des Befestigungssockels gemäß der Fig. 5 bis 9 in der Öffnung 4 der gitterförmigen Struktur 3 der Trägerplatte 2 zu erläutern. Zur Befestigung des Befestigungssockels 6 in einer einzelnen Öffnung 4 der gitterförmigen Struktur 3 wird der Befestigungssockel 6 in der oben in Fig. gezeigten Stellung linear in die Öffnung 4 eingeführt. In dieser Stellung sind die Zwischenseiten 21 parallel zu den Gitterstäben 5 ausgerichtet. Da die Zwischenseiten 21 näher an der Drehachse 26 liegen als die Rastzungen 8, ist in dieser Stellung ein Einführen des Befestigungssockels 6 in die Öffnung 4 ohne weiteres möglich. Ist der Befestigungssockel 6 mit seinen Rastzungen 8 in entsprechender Art und Weise in die Öffnung 4 eingeführt, so wird der Befestigungssockel 6 z.B. durch Einführen eines Schraubenziehers in die Ausnehmunge 10 um die Drehachse 26 so weit gedreht, bis die Rastzungen 8 die Wülste 7 klemmend hintergreifen, womit der Befestigungssockel 6 dann klemmend in der Öffnung 4 befestigt ist. Die entsprechende Endstellung ist in Fig. 10 unten gezeigt.

In dieser Endstellung kann, falls dies als notwendig erachtet wird, noch die entsprechende Spreizschraube 12 in die Ausnehmung 11 eingeschraubt werden, womit die Rastzungen 8 dann rückseitig zusätzlich abgestützt sind um eine besonders feste bzw. stabile Klemmverbindung zwischen Befestigungssockel 6 und gitterförmiger Struktur 3 zu erreichen.

Soll der Befestigungssockel 6 aus der in Fig. 10 unten gezeigten Stellung für die Entnahme des Befestigungssockels 6 aus der Öffnung 4 wieder in die in Fig. 10 oben gezeigte Stellung gebracht werden, so kann der Befestigungssockel 6 entweder in dieselbe Richtung, in der er in die Klemmstellung gedreht wurde, weitergedreht werden. Alternativ ist auch ein Zurückdrehen um die Drehachse 26 in die entsprechende Gegenrichtung möglich. In der in Fig. 10 gezeigten Stellung kann der Befestigungssockel 6 dann wieder einfach aus der Öffnung 4 entnommen werden.

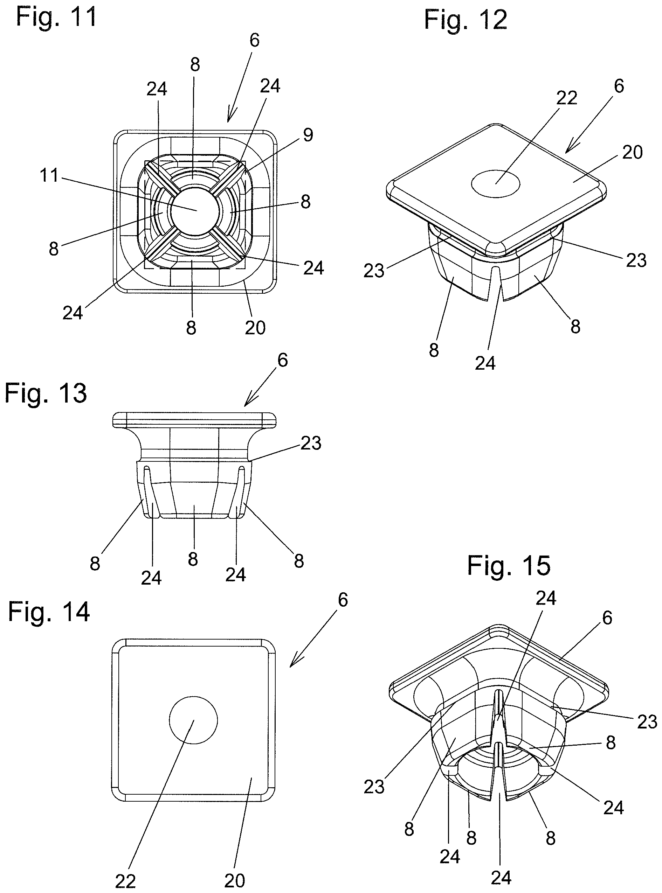

In den Fig. 11 bis 15 ist eine alternative Art eines Befestigungssockels 6 beispielhaft dargestellt. Diese Art der Befestigungssockel 6 ist dazu vorgesehen, dass der Befestigungssockel 6 ausschließlich durch ein, vorzugsweise lineares, Eindrücken des Befestigungssockels 6 in die Öffnung 4 klemmend an der gitterförmigen Struktur 3 befestigt wird. Auch hier sind die vier Rastzungen 8 entlang eines gedachten Quadrats 9 verlaufend angeordnet. Auch diese Rastzungen 8 ergeben eine achsensymmetrische und auch punktsymmetrische Struktur. Zwischen den Rastzungen 8 befinden sich jeweils Spalte 24, welche die elastische Deformation der Rastzungen 8 beim Eindrücken und beim Herausziehen des Befestigungssockels 6 aus der Öffnung 4 in der gitterförmigen Struktur 3 vereinfachen, insbesondere dann, wenn die Rastzungen 8 zwischen den Wülsten 7 eingeschoben werden oder herausgezogen werden müssen. Auch hier weisen die Rastzungen 8 jeweils einen Hinterschnitt 23 auf, mit dem sie die Wülste 7 hintergreifen. In der klemmend angeordneten Stellung des Befestigungssockels 6 in der Öffnung 4 sind die Wülste 7 der Gitterstäbe 5 zwischen der Platte 20 und dem jeweiligen Hinterschnitt 23 der Rastzunge 8 eingeklemmt. Auch bei diesem Ausführungsbeispiel ist eine Ausnehmung 11 zwischen den Rastzungen 8 vorgesehen, in welche bei Bedarf eine Spreizschraube 12 oder ein entsprechender Spreizstift eingeführt werden kann, um die Rastzungen 8 rückseitig abzustützen und damit eine besonders stabile klemmende Befestigung des Befestigungssockels 6 in der Öffnung 4 zu erreichen. Eine Ausnehmung 11 für ein Drehwerkzeug ist hier nicht vorgesehen, da diese Art der Befestigungssockel 6 beim Einführen in und beim Herausnehmen aus der Öffnung 4 nicht gedreht werden.

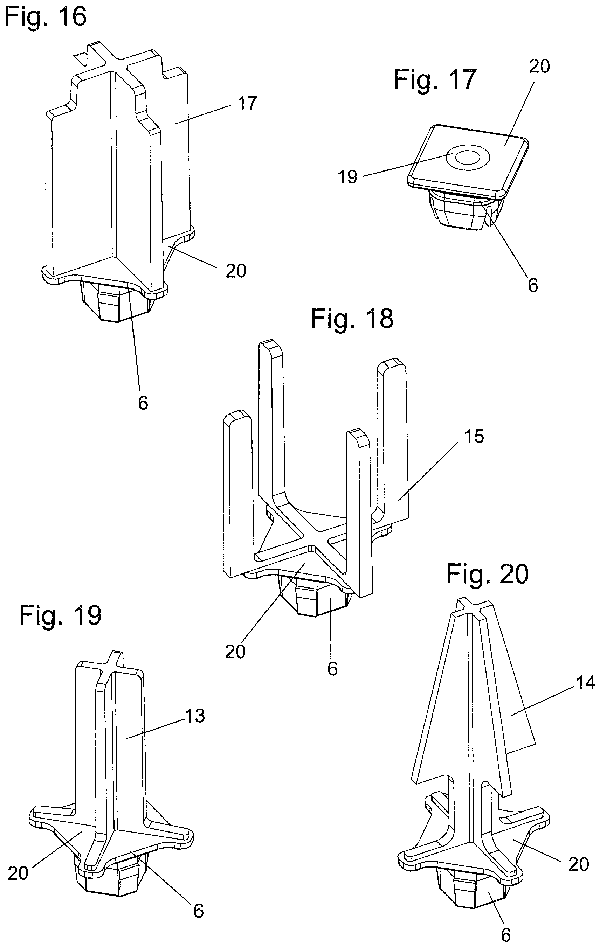

Die Fig. 16 bis 20 zeigen noch einmal erfindungsgemäße Befestigungssockel 6 an denen verschiedene Stützstrukturen beispielhaft angeordnet sind. In den Fig. 16, 18, 19 und 20 sind die jeweiligen Steher 13, 14, 15 und 17 jeweils einstückig angeformt. In Fig. 17 ist noch ein Ausführungsbeispiel eines Befestigungssockels 6 gezeigt, bei dem eine Gewindebuchse 19, vorzugsweise aus Metall vorgesehen ist, an die eine beliebige Stütz- oder sonstige Struktur angeschraubt werden kann.

### Legende zu den Hinweisziffern:

- 1: Trägeranordnung
- 2, 2': Trägerplatte
- 3: gitterförmige Struktur
- 4: Öffnung
- 5: Gitterstab
- 6: Befestigungssockel
- 7: Wulst
- 8: Rastzunge
- 9: gedachtes Quadrat
- 10: Ausnehmung
- 11: Ausnehmung
- 12: Spreizschraube
- 13: gekreuzte Steher
- 14: tannenbaumförmige Steher
- 15: mehrfingerige Steher
- 16: stabförmige Steher
- 17: Stapelsteher
- 18: Griff
- 19: Gewindebuchse
- 20: Platte
- 21: Zwischenseite
- 22: Befestigungsloch
- 23: Hinterschnitt
- 24: Spalt
- 25: Befestigungssockel
- 26: Drehachse

## Patentansprüche

1. Trägeranordnung (1) für die Lagerung und/oder den Transport und/oder die Reinigung von Geschirr oder anderen Gegenständen, wobei die Trägeranordnung (1) zumindest eine Trägerplatte (2) mit zumindest einer gitterförmigen Struktur (3) aufweist, wobei die gitterförmige Struktur (3) von rechteckförmig, insbesondere quadratisch, angeordneten und rechteckförmige, insbesondere quadratische, Öffnungen (4) umschließenden Gitterstäben (5) gebildet ist, und wobei die Trägeranordnung (1) zumindest einen Befestigungssockel (6) aufweist, welcher klemmend an der gitterförmigen Struktur (3) befestigbar oder befestigt ist, wobei der Befestigungssockel (6) in einer einzelnen der rechteckförmigen, insbesondere quadratischen, Öffnungen (4) klemmend befestigbar oder befestigt ist und die die Öffnungen (4) umschließenden Gitterstäbe (5) jeweils zumindest eine Wulst (7) zur klemmenden Befestigung des Befestigungssockels (6) in der Öffnung (4) aufweisen und der Befestigungssockel (6) eine Platte (20) aufweist, wobei zwischen Hinterschnitten (23) von Rastzungen (8) des Befestigungssockels (6) und der Platte (20) die Wulst (7), vorzugsweise spielfrei, einklemmbar oder eingeklemmt ist, **dadurch gekennzeichnet, dass** der Befestigungssockel (6) vier paarweise einander gegenüberliegende Rastzungen (8) aufweist und die Hinterschnitte (23) aller Rastzungen (8) des Befestigungssockels (6) gleich weit von der Platte (20) distanziert sind und dass alle Wülste (7) der Trägerplatte (2) in einer gemeinsamen Ebene enden.

2. Trägeranordnung (1) nach Anspruch 1, **dadurch gekennzeichnet, dass** jede Wulst (7) auf einer, eine Oberfläche der Trägerplatte (2) bildenden Seite der Gitterstäbe (5) ausgebildet ist.

3. Trägeranordnung (1) nach Anspruch 1 oder 2, **dadurch gekennzeichnet, dass** die Rastzungen (8) zur Befestigung des Befestigungssockels (6) in der Öffnung (4) jeweils die Wulst (7) eines der die Öffnung (4) umschließenden Gitterstäbe (5) hintergreift und/oder dass die paarweise einander gegenüberliegenden Rastzungen (8), vorzugsweise in Richtung voneinander weg, elastisch vorgespannt sind.

4. Trägeranordnung (1) nach einem der Ansprüche 1 bis 3, **dadurch gekennzeichnet, dass** zur Vereinfachung einer elastischen Deformation der Rastzungen (8) zwischen zwei jeweils zueinander benachbarten Rastzungen (8), vorzugsweise jeweils, ein Spalt (24) angeordnet ist.

5. Trägeranordnung (1) nach einem der Ansprüche 1 bis 4, **dadurch gekennzeichnet, dass** die Rastzungen (8) zumindest bereichsweise entlang der Seiten eines gedachten Rechtecks, insbesondere Quadrats (9), verlaufen.

6. Trägeranordnung (1) nach Anspruch 5, **dadurch gekennzeichnet, dass** zwischen zwei jeweils benachbarten Rastzungen (8) sich jeweils eine Zwischenseite (21) des Befestigungssockels (6) befindet, wobei die Zwischenseiten (21) in einem geringeren Abstand als die Rastzungen (8) von einer Drehachse (26) des Befestigungssockels (6) angeordnet sind.

7. Trägeranordnung (1) nach einem der Ansprüche 1 bis 6, **dadurch gekennzeichnet, dass** die Trägerplatte (2) und/oder der Befestigungssockel (6) zumindest zum Teil, vorzugsweise vollständig, aus Kunststoff ausgebildet ist bzw. sind.

8. Trägeranordnung (1) nach einem der Ansprüche 1 bis 7, **dadurch gekennzeichnet, dass** im in einer der Öffnungen (4) befestigten Zustand des Befestigungssockels (6) die Gitterstäbe (5) auf einer Seite der gitterförmigen Struktur (3) über den Befestigungssockel (6) überstehen.

9. Trägeranordnung (1) nach einem der Ansprüche 1 bis 8, **dadurch gekennzeichnet, dass** der Befestigungssockel (6), vorzugsweise im Bereich zwischen den Rastzungen (8), zumindest eine Ausnehmung (10) für den Eingriff eines Drehwerkzeugs, vorzugsweise Schraubenziehers, aufweist, und/oder dass ein Spreizstift oder eine Spreizschraube (12) in einer Ausnehmung (11) des Befestigungssockels (6) zwischen Rastzungen (8) des Befestigungssockels (6) aufgenommen ist.

10. Trägeranrodnung (1) nach einem der Ansprüche 1 bis 9, **dadurch gekennzeichnet, dass** der Befestigungssockel (6) eine zerstörungsfrei lösbare Verbindungseinrichtung, vorzugsweise in Form einer Gewindebuchse (19), zur Befestigung einer Stützstruktur oder einer sonstigen Struktur am Befestigungssockel (6) aufweist.

11. Trägeranordnung (1) nach einem der Ansprüche 1 bis 10, **dadurch gekennzeichnet, dass** der Befestigungssockel (6) ausschließlich durch ein, vorzugsweise lineares, Eindrücken des Befestigungssockels (6), vorzugsweise seiner Rastzungen (8), in die Öffnung (4) klemmend an der gitterförmigen Struktur (3) befestigbar oder befestigt ist.

12. Trägeranordnung (1) nach einem der Ansprüche 1 bis 10, **dadurch gekennzeichnet, dass** der Befestigungssockel (6), vorzugsweise ausschließlich, durch ein, vorzugsweise lineares, Einführen des Befestigungssockels (6), vorzugsweise seiner Rastzungen (8), in die Öffnung (4) und ein anschließendes Drehen des Befestigungssockels (6) in der Öffnung (4) klemmend an der gitterförmigen Struktur (3) befestigbar oder befestigt ist.

## Claims

1. Carrier arrangement (1) for storing and/or transporting and/or cleaning dishes or other articles, wherein the carrier arrangement (1) comprises at least one carrier plate (2) having at least one lattice-shaped structure (3), wherein the lattice-shaped structure (3) is formed by lattice bars (5) which are arranged in a rectangular shape, in particular in a square shape, and surround rectangular, in particular square, openings (4), and wherein the carrier arrangement (1) comprises at least one fastening base (6) which can be or is clampingly fastened to the lattice-shaped structure, wherein the fastening base (6) can be or is clampingly fastened in a single one of the rectangular, in particular square, openings (4), and the lattice bars (5) surrounding the openings (4) each have at least one bead (7) for clampingly fastening the fastening base (6) in the opening (4), and the fastening base (6) has a plate (20), wherein the bead (7) can be or is clamped, preferably without play, between undercuts (23) of latching tongues (8) of the fastening base (6) and the plate (20), **characterized in that** the fastening base (6) has four latching tongues (8) located opposite one another in pairs, and the undercuts (23) of all the latching tongues (8) of the fastening base (6) are at an equal distance from the plate (20), and **in that** all the beads (7) of the carrier plate (2) end in a common plane.

2. Carrier arrangement (1) according to claim 1, **characterized in that** each bead is formed on a side of the lattice bars (5) that forms a surface of the carrier plate (2) .

3. Carrier arrangement (1) according to claim 1 or 2, **characterized in that** the latching tongues (8) for fastening the fastening base (6) in the opening (4) each engage behind the bead (7) of one of the lattice bars (5) surrounding the opening (4), and/or **in that** the latching tongues (8) located opposite one another in pairs are elastically biased, preferably in the direction away from one another.

4. Carrier arrangement (1) according to one of claims 1 to 3, **characterized in that**, to facilitate elastic deformation of the latching tongues (8), a gap (24) is arranged preferably between each two respectively adjacent latching tongues (8).

5. Carrier arrangement (1) according to one of claims 1 to 4, **characterized in that** the latching tongues (8) run, at least in some regions, along the sides of an imaginary rectangle, in particular square (9).

6. Carrier arrangement (1) according to claim 5, **characterized in that** an intermediate side (21) of the fastening base (6) is in each case located between two respectively adjacent latching tongues (8), wherein the intermediate sides (21) are arranged at a smaller distance than the latching tongues (8) from an axis of rotation (26) of the fastening base (6).

7. Carrier arrangement (1) according to one of claims 1 to 6, **characterized in that** the carrier plate (2) and/or the fastening base (6) is or are at least partially, preferably completely, made of plastic.

8. Carrier arrangement (1) according to one of claims 1 to 7, **characterized in that**, when the fastening base (6) is fastened in one of the openings (4), the lattice bars (5) protrude beyond the fastening base (6) on one side of the lattice-shaped structure (3).

9. Carrier arrangement (1) according to one of claims 1 to 8, **characterized in that** the fastening base (6) has, preferably in the region between the latching tongues (8), at least one recess (10) for engagement of a rotary tool, preferably a screwdriver, and/or **in that** an expanding pin or an expanding screw (12) is received in a recess (11) of the fastening base (6) between latching tongues (8) of the fastening base (6).

10. Carrier arrangement (1) according to one of claims 1 to 9, **characterized in that** the fastening base (6) has a non-destructively releasable connecting device, preferably in the form of a threaded bushing (19), for fastening a support structure or some other structure to the fastening base (6).

11. Carrier arrangement (1) according to one of claims 1 to 10, **characterized in that** the fastening base (6) can be or is clampingly fastened to the lattice-shaped structure (3) solely by pressing, preferably in a linear fashion, the fastening base (6), preferably the latching tongues (8) thereof, into the opening (4).

12. Carrier arrangement (1) according to one of claims 1 to 10, **characterized in that** the fastening base (6) can be or is clampingly fastened to the lattice-shaped structure (3) preferably solely by introducing, preferably in a linear fashion, the fastening base (6), preferably the latching tongues (8) thereof, into the opening (4) and then rotating the fastening base (6) in the opening (4).

## Revendications

1. Dispositif de support (1) pour le stockage et/ou le transport et/ou le nettoyage de vaisselle ou d'autres objets, le dispositif de support (1) étant muni d'au moins une plaque de support (2) avec au moins une structure en forme de grille (3), la structure en forme de grille (3) étant formée de baguettes de grille (5) disposées de façon rectangulaire, en particulier de façon carrée, et entourant des ouvertures (4) rectangulaires, en particulier carrées, et le dispositif de support (1) étant muni d'au moins un socle de fixation (6) qui peut être fixé ou qui est fixé de façon serrée à la structure en forme de grille (3), le socle de fixation (6) pouvant être fixé ou étant fixé de façon serrée dans une seule des ouvertures (4) rectangulaires, en particulier carrées, et les baguettes de grille (5) entourant les ouvertures (4) étant munies chacune d'au moins un bourrelet (7) pour fixer de façon serrée le socle de fixation (6) dans l'ouverture (4), et le socle de fixation (6) étant muni d'une plaque (20), le bourrelet (7) pouvant être serré ou étant serré, de préférence sans jeu, entre la plaque (20) et des contre-dépouilles (23) de languettes d'encliquetage (8) du socle de fixation (6), **caractérisé en ce que** le socle de fixation (6) est muni de quatre languettes d'encliquetage (8) se faisant face par paires et les contre-dépouilles (23) de toutes les languettes d'encliquetage (8) du socle de fixation (6) sont à égale distance de la plaque (20), et **en ce que** tous les bourrelets (7) de la plaque de support (2) se terminent dans un plan commun.

2. Dispositif de support (1) selon la revendication 1, **caractérisé en ce que** chaque bourrelet (7) est formé sur un côté des baguettes de grille (5) formant une surface de la plaque de support (2).

3. Dispositif de support (1) selon la revendication 1 ou 2, **caractérisé en ce que** les languettes d'encliquetage (8) pour la fixation du socle de fixation (6) dans l'ouverture (4) s'engagent chacune derrière le bourrelet (7) de l'une des baguettes de grille (5) entourant l'ouverture (4), et/ou **en ce que** les languettes d'encliquetage (8) se faisant face par paires sont précontraintes élastiquement, de préférence dans la direction opposée à l'autre.

4. Dispositif de support (1) selon l'une des revendications 1 à 3, **caractérisé en ce qu'**une fente (24) est disposée entre deux languettes d'encliquetage (8) adjacentes, de préférence entre chacune d'elles, afin de simplifier une déformation élastique des languettes d'encliquetage (8).

5. Dispositif de support (1) selon l'une des revendications 1 à 4, **caractérisé en ce que** les languettes d'encliquetage (8) s'étendent, au moins par zones, le long des côtés d'un rectangle imaginaire, en particulier d'un carré (9).

6. Dispositif de support (1) selon la revendication 5, **caractérisé en ce qu'**un côté intermédiaire (21) du socle de fixation (6) est situé entre deux languettes d'encliquetage (8) adjacentes, les côtés intermédiaires (21) étant disposés à une distance plus petite d'un axe de rotation (26) du socle de fixation (6) que les languettes d'encliquetage (8).

7. Dispositif de support (1) selon l'une des revendications 1 à 6, **caractérisé en ce que** la plaque de support (2) et/ou le socle de fixation (6) sont réalisés au moins en partie, de préférence en totalité, en matière plastique.

8. Dispositif de support (1) selon l'une des revendications 1 à 7, **caractérisé en ce que**, à l'état fixé du socle de fixation (6) dans l'une des ouvertures (4), les baguettes de grille (5) saillent d'un côté de la structure en forme de grille (3) au-dessus du socle de fixation (6).

9. Dispositif de support (1) selon l'une des revendications 1 à 8, **caractérisé en ce que** le socle de fixation (6) est muni, de préférence dans la zone située entre les languettes d'encliquetage (8), d'au moins un évidement (10) pour l'engagement d'un outil rotatif, de préférence d'un tournevis, et/ou **en ce qu'**un goujon à expansion ou une vis à expansion (12) est reçu dans un évidement (11) du socle de fixation (6) entre les languettes d'encliquetage (8) du socle de fixation (6).

10. Dispositif de support (1) selon l'une des revendications 1 à 9, **caractérisé en ce que** le socle de fixation (6) est muni d'un dispositif de liaison pouvant être libéré sans destruction, de préférence sous la forme d'une douille filetée (19), pour la fixation au socle de fixation (6) d'une structure de soutien ou d'une autre structure.

11. Dispositif de support (1) selon l'une des revendications 1 à 10, **caractérisé en ce que** le socle de fixation (6) peut être fixé ou est fixé de façon serrée sur la structure en forme de grille (3) exclusivement en pressant dans l'ouverture (4), de préférence de façon linéaire, le socle de fixation (6), de préférence ses languettes d'encliquetage (8).

12. Dispositif de support (1) selon l'une des revendications 1 à 10, **caractérisé en ce que** le socle de fixation (6) peut être fixé ou est fixé de façon serrée sur la structure en forme de grille (3), de préférence exclusivement, en insérant dans l'ouverture (4), de préférence de façon linéaire, le socle de fixation (6), de préférence ses languettes d'encliquetage (8), et en faisant ensuite tourner le socle de fixation (6) dans l'ouverture (4).
